# FASCICULE DE BREVET EUROPEEN

(11) **EP 1 244 367 B1**
(45) Date de publication et mention de la délivrance du brevet: **17.08.2005**
(21) Numéro de dépôt: 00981640.6
(22) Date de dépôt: 23.11.2000
(51) Int. Cl.: A41H 1/02

(54) **PROCEDE POUR L'IDENTIFICATION ET LA CLASSIFICATION ANTHROPOMORPHIQUE A USAGE MILITAIRE ET CIVIL**
VERFAHREN ZUR IDENTIFIZIERUNG UND ANTROPOMORPHISCHEN KLASSIFIZIERUNG FÜR DEN MILITÄRISCHEN SOWIE ZIVILEN GEBRAUCH
PROCEDE POUR L'IDENTIFICATION ET LA CLASSIFICATION ANTHROPOMORPHIQUE A USAGE MILITAIRE ET CIVIL

(30) Priorité: 24.11.1999 IT PC990043
(43) Date de publication de la demande: 02.10.2002
(73) Titulaire: Cad Modelling Technology Mission S.r.l., 50121 Firenze (IT)
(72) Inventeur: DUHAMEL, Elisabeth, I-50121 Firenze (IT)
(86) Numéro de dépôt international: PCT/IT2000/000475
(87) Numéro de publication internationale: WO 2001/037692

(56) Documents cités:
- EP-A- 0 524 119
- EP-A- 0 554 647
- WO-A-95/04975
- US-A- 4 406 544
- US-A- 5 163 006

## Description

### Obiet de l'invention et domaine d'application.

La présente invention concerne un procédé apte à l'identification et classification automatique des "conformations anthropométriques"; par le procédé de la présente invention on parvient :
a. au relèvement automatique du volume du corps humain ;
b. à la comparaison dudit volume avec des volumes des normotypes codifiés selon la forme dudit volume ;
c. à la conséquente classification dudit volume selon des critères qui caractérisent normotypes codifiés.

Les domaines d'application de la présente invention concernent aussi bien l'industrie de l'habillement que tous les autres domaines pour lesquels la volumétrie du corps humain a une importance déterminante vis-à-vis du résultat final, comme par exemple dans le domaine militaire.

Dans ce dernier domaine, en ettet, il est fondamental de répondre à l'exigence du bien-aller correcte et confortable des uniformes pour des raisons, normalement en conflit, de sécurité et de fonctionnalité.

Toujours dans le domaine militaire, le procédé de la présente invention trouvent leurs applications là où il est important d'optimiser le rapport entre les paramètres corporels de l'utilisateur (poids, volume, forme) et l'équipement ou le matériel de travail ou de combat (longueur des sangles pour le support d'armes en bandoulière, cabines, habitacles, etc).

D'autres applications possibles de la présente invention investissent l'industrie des transports dans laquelle, afin de projeter des moyens de transport aussi bien civils que militaires, tels que voitures, Jeep, camions, chars d'assaut et avions, la connaissance des différentes formes du corps humain à parité de volume permet de réaliser les habitacles et les sièges de manière ergonomique. On trouve les mêmes exigences dans l'industrie de l'ameublement, où des projets de mobilier de soutien tels que chaises, canapés et fauteuils peuvent être optimisés en tenant compte des formes réelles volumétriques statistiquement déterminées des utilisateurs.

### Avantages de l'invention

Un grave et typique inconvénient vérifiable, comme exemple non limitatif, dans l'industrie de l'habillement, dérive de l'énorme quantité de pièces d'habillement invendue à cause de leur bien-aller incorrect.

En effet, des millions de pièces d'habillement introduites sur le marché sont fabriquées selon les morphologies idéales, lesquelles diffèrent, toutefois, de manière considérable des réelles configurations anthropométriques : le.résultat de ladite discordance résulte en ce que le produit est généralement inapte à habiller les utilisateurs. Cette grave et typique déséconomie de l'industrie de l'habillement provient du fait que les morphologies idéales humaines, classifiées selon un système de tailles, drop, ou indications du type S, M, L, XL, etc... , selon les normes ISO, sont seulement représentatives du volume du corps humain mais non, à parité de volume, de sa forme, là où pour forme on entend la distribution des mesures d'ellipsoïdes le long du développement en hauteur du volume du corps humain ; les tailles, réglées par les normes ISO 3635, ne sont pas suffisantes pour une construction correcte des vêtements ; dans ce but la norme ISO 8559 permet de fournir des statistiques anthropométriques pour une construction plus correcte des vêtements.

En plus, le bien-aller des pièces d'habillement doit être essayé sur le corps en mouvement, les articulations étant les points critiques pour modeler le vêtement.

La méthodologie réellement utilisée pour le projet et la production en masse des pièces d'habillement est, comme susmentionné, basée sur le système de tailles et sur le développement des mesures linéaires des périmètres des parties constituant la pièce, les mesures étant variables dans un écart dont les extrémités sont calculées en fonction du type de population à habiller, population pour laquelle les différences entre les tailles adjacentes sont de l'ordre de 2-4 cm.

Un telle formulation, comme on peut le voir sur les tableaux des tailles reportés dans les catalogues par correspondance ou, par exemple, dans le qualifié "Book of the Ostend Institute of Berlin", utilisé de préférence pour le contrôle anthropométrique allemand, implique par approximation que le tour de thorax, le tour de taille, le tour des hanches et autres mesures de référence fondamentales établies par les normes ISO grandissent de la valeur minimum à la maximum avec la même proportion.

Cette méthodologie implique donc que le corps humain soit assimilé à un "ballon élastique" de forme anatomique lequel, de manière analogue au ballon sphérique, se gonfle et s'étend uniformément et proportionnellement en toutes ses parties, c'est-à-dire qu'à une augmentation du tour du thorax correspond une nécessaire et proportionnelle augmentation du tour de taille et des autres ellipsoïdes.

En pratique, le corps humain n'augmente pas selon cette loi et, comme on sait que parmi certaines catégories d'âge (par exemple pendant le passage de l'adolescent à l'adulte) un tel modèle peut être adopté pour approcher le mode de développement du corps humain, à partir d'un certain âge, par contre, on vérifie une augmentation indépendante et non mise en corrélation des différentes parties du corps, on peut donc vérifier que le tour de taille augmente là où le tour du thorax reste constant : on vérifie donc un changement de conformation anthropométrique dans le sens que l'augmentation du volume comporte une variation de la forme du corps, puisque les ellipsoïdes qui caractérisent le volume changent dans le rapport de corrélation. Ledit problème, bien qu'il concerne l'industrie de l'habillement, est inhérent à la production industrielle de grande séries de pièces ; dans le passé il était réglé par les maisons de couture artisanales par des adaptations de la pièce au corps de l'utilisateur, afin de respecter sa réelle conformation volumétrique, en tenant compte aussi des autres paramètres affectant le bien-aller du vêtement, comme par exemple les caractéristiques mécaniques du tissu et son élasticité.

Il en résultait que, à parité des mesures standard (taille) le vêtement nécessitait des ajustements selon les caractéristiques du tissu et de la forme particulière du corps, c'est-à-dire de la réelle conformation anthropométrique de l'utilisateur.

Une première solution partielle à ce problème est représentée par l'adoption pour la fabrication des vêtements de mannequins anthropométriques du type divulgués en EP 0634114 (Cad-Modelling).

Les formes desdits mannequins se fondent sur des statistiques anthropométriques de base, élaborées par des moyens informatiques et prenant en considération des valeurs moyennes pondérés des volumes du corps humain.

Lesdits mannequins anthropométriques articulés, qui correspondent aux volumes et proportions représentant les valeurs moyennes des conformations anthropométriques, adoptent la méthodologie de classification qui est à la base de la présente invention.

Afin d'optimiser le procédé de production des pièces d'habillement, ou pour toutes les autres applications susmentionnées dans d'autres secteurs de l'industrie, pour optimiser les formes finales des produits, la présente invention utilise une "numérisation" des conformations desdits mannequins articulés.

Lesdites représentations numériques des conformations sont ensuite mémorisées afin de construire une base de données qui consent de confronter ladite représentation numérique avec la codification numérique de la forme anatomique à habiller:
la forme anatomique est relevée et numérisée par le procédé de la revendication principale. On individualise, de cette manière, la conformation exacte de l'utilisateur représentable selon la classification correcte de la conformation anthropométrique et donc, par conséquent, on individualise le bien-aller correct du vêtement.

### Etat de la technique antérieure

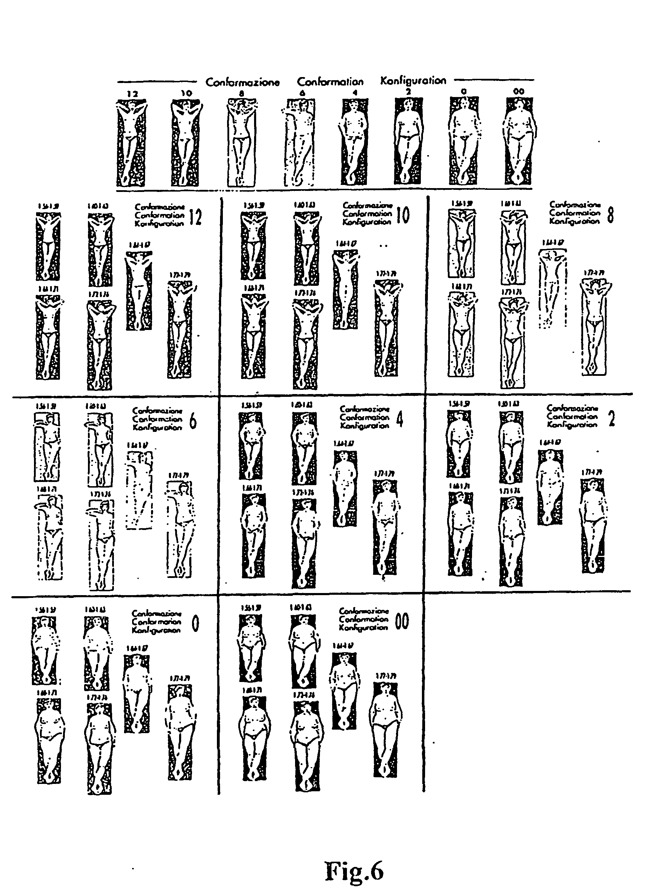

A la base du fonctionnement de la présente invention, comme susmentionné, se trouve un système de classification des formes humaines, comme divulgué par EP 0634114 ; selon ledit système de classification, les formes peuvent être distinguées en six classes morphologiques pour l'homme, sept pour la femme et vingt pour l'enfant/adolescent (Fig.6).

La théorie à la base dudit système de classification est aussi divulguée dans "Bringing Anthropometric Data into the 20^{th} Century" de Manuel Gaetan Ph. D., Apparel Manufacturer, Nov.1989.

Un autre support théorique à la base de la présente invention est donné par les méthodes de représentation tridimensionelle des formes, comme elles sont décrites dans "Algorithms for graphic and images processing" de Theo Pavlidis, Springer-Verlag, Berlin, particulièrement au chapitre 17, pages 77-392.

Le problème du relèvement des caractéristiques géométriques du corps humain a également été traité par JP-A-6104430 dans lequel on décrit un système pour produire des configurations pour l'industrie de l'habillement, consistant en l'élaboration numérique des "silhouettes" d'une personne prises au moyen d'un système vidéo ; les limites du système sont inhérentes au système de relèvement qui, étant constitué de deux images orthogonales, de face et de profil, ne permettent pas une représentation volumétrique précise des formes humaines. De plus, le système se limite à mesurer le corps de manière identique aux opérations manuelles correspondantes, sans prétentions de sa classification anthropométrique, par absence d'une référence anthropométrique standard.

Donc, même si on résout le problème de la mesure sans contact, les enseignements divulgués par JP-A-6104430 ne résolvent pas le problème de la rationalisation de la reproduction industrielle afin de réduire le gaspillage mais ils résolvent seulement le problème du relèvement des mesures sans le contact ennuyeux lors de l'utilisation d'un mètre de couture pour les relevés manuels.

Dans EP 0524119 (Telmat) sont divulgués un procédé et un dispositif pour l'identification et la représentation de sujets humains. Les limites vérifiées pour une telle divulgation, par rapport au procédé et à l'appareil de la présente invention, consistent en ce que la représentation tridimensionnelle en forme numérique est obtenue au moyen de procédés de calcul basé sur la simulation à réseaux neurales (systèmes d'auto-apprentissage), ce qui comporte des procédés de calcul complexes et coûteux et l'utilisation de calculateurs onéreux utilisant aussi le calcul parallèle, où selon le procédé de la présente invention sont utilisés des ordinateurs commerciaux sans nécessité de logiciel compliqué.

US 4 406 544 se propose de mesurer les corps des personnes humaines sans toucher les personnes et de mémoriser les données; aucun autre problème n'est résolu avec ce procédé et l'appareil;

WO 95/04975 se propose de prendre un groupe de personnes de la même taille, de le mesurer selon un procédé automatique et de réaliser un modèle de taille qui soit le plus satisfaisant pour ladite taille.

La présente demande de brevet se propose de créer une base de données de formes volumétriques divisées en conformations anthropométriques différentes pour les hommes, les femmes et les enfants, de mesurer les corps humains et, en fonction des mesures effectuées, d'attribuer une conformation anthropométrique à ladite anatomie.

Un autre problème résolu par la présente invention est celui d'utiliser une seule télécaméra pour obtenir l'enchevêtrement de la forme du volume du sujet examiné, en faisant tourner ledit sujet de 360° au moyen d'une estrade roulante synchronisée avec le système d'acquisition des vues : cela est possible en fixant une référence du début de la rotation et en synchronisant la rotation et la scansion avec le module d'acquisition des données provenant de la télécaméra, là où les techniques d'acquisition des images de l'état de la technique antérieure fondent le procédé de calcul sur deux vues, respectivement de face et de profil ; le même état de la technique antérieure prévoit pour les réalisation à plusieurs vues d'utiliser plusieurs caméras, ou des télécaméras tournantes avec une conséquente variation de la profondeur de l'image ; en plus, dans le cas de tournage consécutif et/ou continu, les caméras ne sont pas référées à une référence de position initiale de la scansion, avec une conséquente imprécision ou même l'impossibilité du relèvement.

La réalisation préférentielle de la présente invention pourvoit à la solution des problèmes exposés au moyen d'un enregistrement de vues du sujet à relever à partir d'une position de référence, dite position de référence constituant le début de la scansion des vues, lesdites vues étant comprises entre un minimum de 90 et un maximum de 92 et étant prises en succession dans un temps de 10 secondes environ.

Au moyen dudit procédé on élimine la nécessité de calculer au moyen d'un simulateur des réseaux neurales le contour de la figure humaine, comme il est au contraire nécessaire dans le cas de EP 0524119.

Ultérieurement, selon la méthode de la technique antérieure lesdits contours sont relevés par des approximations successives basées sur un nombre de vues non précisé (au moins une) et en utilisant une méthode de classification et représentation des formes humaines au moyen d'un dispositif de prise de vues planaire, fondées sur un procédé d'apprentissage à réseaux neurales : on a pour conséquence des imprécisions évidentes dans la reconnaissance et la classification desdites formes en fonction soit du nombre de caméras utilisées, soit aussi de leur disposition, soit encore du nombre de vues relevées par chacun des relèvements.

Afin de relever le profil du corps, le système divulgué par EP 0524119, au moins dans sa réalisation préférentielle, utilise deux télécaméras orthogonales et fonde ainsi la construction du volume réel de manière approximative : cela comporte l'impossibilité ou l'insuffisante précision dans la détermination et la caractérisation de la forme dudit volume. En partant, au contraire, de la susdite théorie des conformations anthropométriques, la présente invention permet d'arriver à un procédé et un appareil pour une correcte identification des formes et leur classification anthropométrique précise selon les susmentionnées classes morphologiques, au moyen de scansion rotatoire du sujet à partir d'une position de référence et avec acquisition numérique synchronisée des vues, lesdites vues étant acquises avec un taux entre 90 et 92 pour chaque tour complet de 10 secondes environ. Les classes morphologiques identifient des structures de forme et de volume, appelées normotypes, qui sont représentées graphiquement de façon réaliste. De plus, comme on voit dans les tableaux I et II, lesdites classes, appelées bases, sont cohérentes avec le système ISO de taille : ceci simplifie l'auto identification et ne bouleverse pas l'existante standardisation des normes ISO adoptées par l'industrie du secteur.

Chacun desdits normotypes est caractérisé par un volume, duquel sont extrapolées une série d'ellipsoïdes, elles-mêmes relevées en parties et en points de référence anatomiques fondamentales pour la construction d'un vêtement.

L'observation fondamentale est que ces mesures ont parmi elles un rapport bien précis, en restant constantes pour différentes statures et en oscillant de toute façon autour d'une valeur qui résulte être la moyenne statistique d'une particulière conformation anthropométrique : cette conformation représente, donc, la valeur la plus représentative de ce volume particulier, en ayant ces proportions spécifiques (distribution des mesures le long du volume du corps).

La gradation, ou encore le changement des rapports des mesures, regardera uniquement une amplitude limitée par rapport à la valeur centrale ; pour les différentes hauteurs possibles à l'intérieur de cette conformation anthropométrique spécifique, dit modèle de base, la pièce d'habillement relative subira des interventions qui se réfèrent seulement aux simples changements de longueur d'éléments ne caractérisant pas le bien-aller et la silhouette.

En conclusion, une conformation anthropométrique déterminée est statistiquement vérifiable dans un domaine limité de hauteurs et dans un domaine de tailles encore plus limité. Un changement de mesures qui soit supérieur aux amplitudes prévues dans les tableaux I et II comporte le changement de conformation et le modèle relatif ne peut plus être obtenu par une simple gradation. Une fois individualisée une conformation anthropométrique déterminée, une de ces possible amplitudes volumétriques qui garde les rapports d'appartenance à cette classe est statistiquement justifiée seulement pour une mesure moyenne et elle prévaut dans un groupe limité de statures ; un écart supérieur comporte un changement de proportions parmi les parties fondamentales du corps et, donc, un changement de conformation anthropométrique : un individu, pendant la croissance, ne passe pas avec continuité d'une mesure à l'autre mais, dans certaines phases particulières de son évolution morphologique, il change de conformation anthropométrique.

Ces considérations permettent la constitution d'une base de données limitée qui simplifie la comparaison avec les données numérisées acquises par la scansion des vues du sujet qui tourne et rend moins complexe le logiciel d'identification.

### Description de l'invention

L'invention présente peut être mieux comprise au moyen des figures :

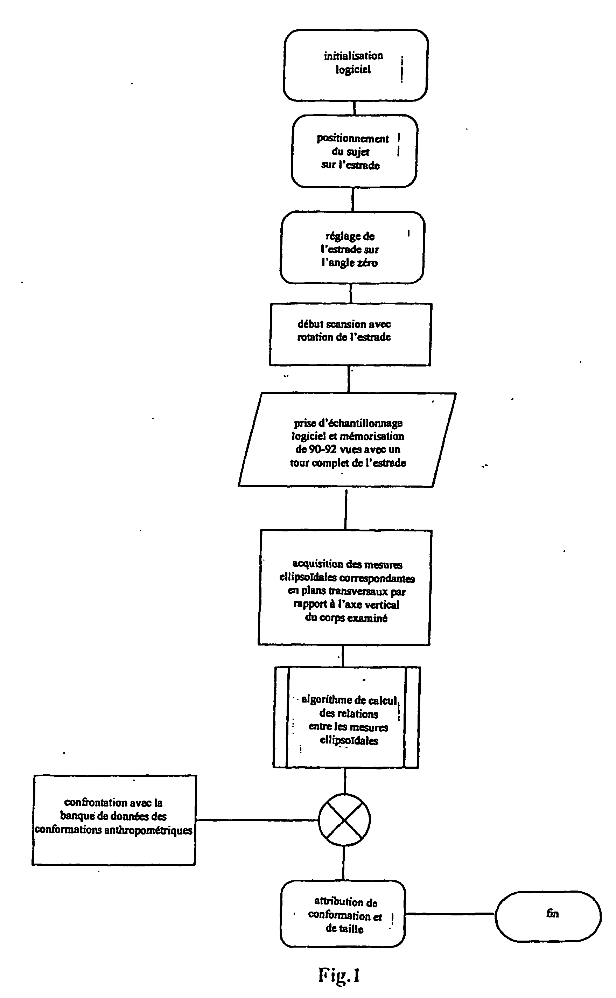

La fig.1 représente un flow chart du procédé ;

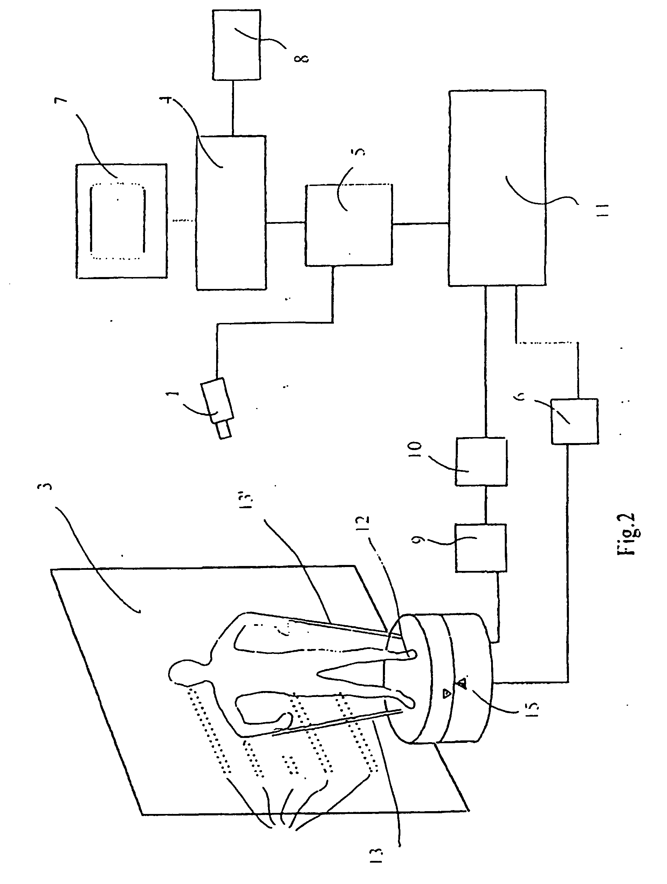

La fig.2 schématise l'appareil qui réalise le procédé inventé ;
La fig.3 montre le masque d'entrée des données du sujet à relever et de sortie des données du relèvement ;
La fig.4 illustre comment mesurer les ellipsoïdes pour des plans inclinés d'une façon paramétrique par rapport à l'axe vertical du sujet de la scansion ;

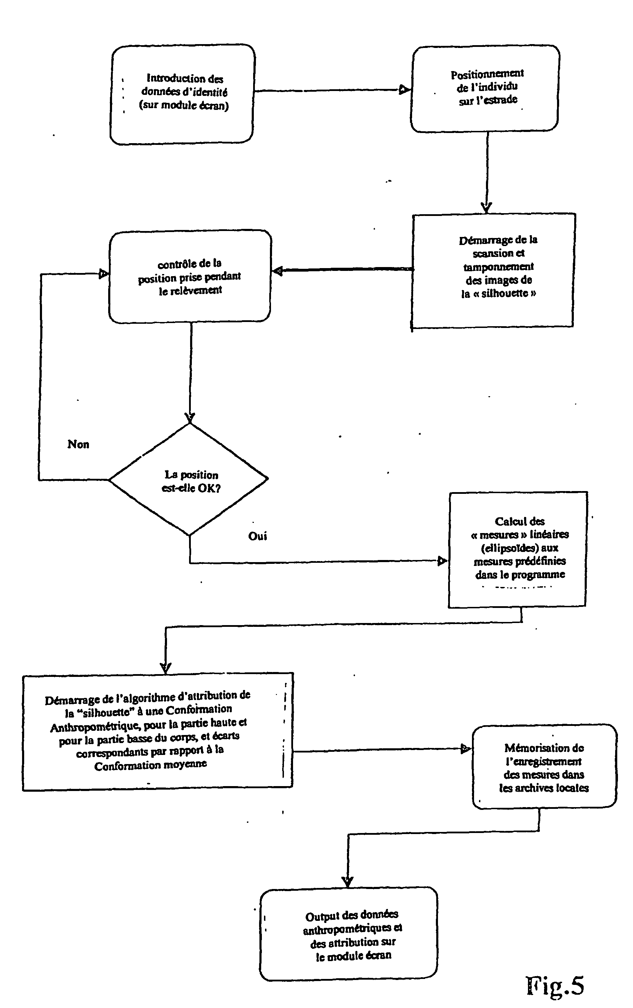

La fig.5 est un diagramme du flux des opérations à effectuer à l'acte du relèvement ;
La fig.6 montre les conformations anthropométriques selon la classification des formes des volumes en relation aux hauteurs, représentées en forme numérique dans la base de données ;

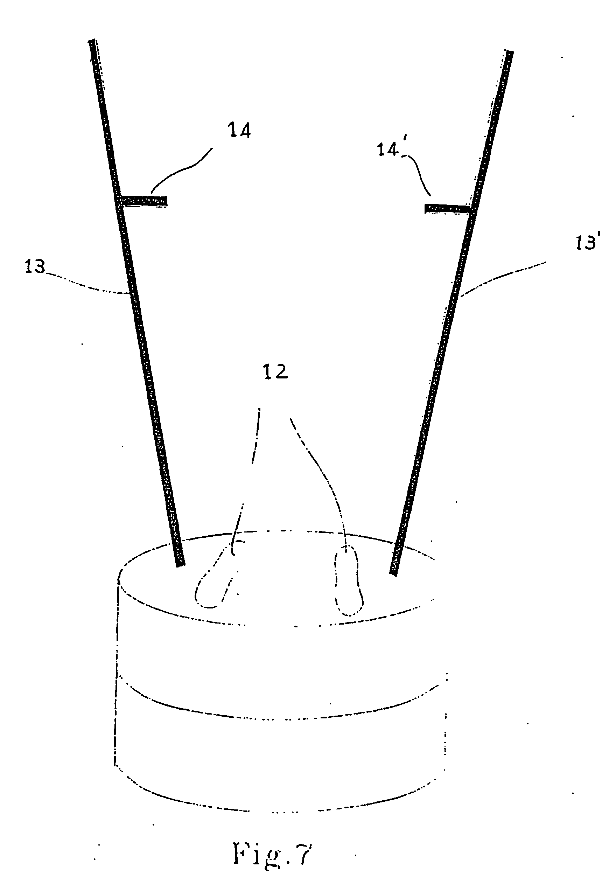

La fig.7 montre les parties qui constituent le système tournant (estrade).

Le procédé de la présente invention se fonde sur l'acquisition numérique des images (vues) obtenues par la scansion d'un sujet, qu'il soit un homme, une femme ou un enfant. Les informations numériques représentent des mesures linéaires qui regardent des parties du corps humain et sont constituées essentiellement par des longueurs de segments linéaires (membres, épaules, hauteur) ou par des ellipsoïdes (tour de poitrine, tour du cou, hanches, etc.).

Lorsque elles sont acquises, lesdites mesures sont mises en relation entre elles, et par comparaisons internes avec la base de données des conformations, elles aussi basées sur des mesures linéaires conformes, elles fournissent une classification des anatomies selon des critères qui caractérisent les volumes et leur forme, comme il a été décrit précédemment. En définitive, donc, le procédé selon la revendication 1 est susceptible de fournir les mesures du corps humain et une classification du corps selon des normotypes dont les paramètres caractéristiques sont précédemment emmagasinés dans la mémoire sous forme de données numériques mémorisées dans la base de données. Le principe du relèvement est centré, comme on a dit, sur l'analyse d'un minimum de 90 à un maximum de 92 silhouettes, constituées d'autant de vues du corps humain ; le sujet sous relèvement est fait tourner autour de son axe longitudinal de 360 degrés ; le nombre de vues relevées d'un minimum de 90 à un maximum de 92 est obtenu comme optimisation entre l'exigence d'avoir le nombre majeur desdites vues pour pouvoir, par intégration, obtenir le volume avec un degré élevé de précision, et l'exigence de minimiser la mémoire RAM, laquelle garde les données numériques relatives aux dites vues. Les vues sont analysées en relation au contour du corps.

Chaque silhouette est obtenue par contraste entre le corps même et un panneau (3) à luminosité homogène posé derrière lui. Le système de relèvement prévoit donc un point de relèvement fixe, constitué par une caméra (1), par rapport au sujet à relever, qui, au contraire, tournera sur lui-même : cela évite des variations de la profondeur du champ comme dans le cas de télécaméras et panneau qui tournent autour du sujet et est, de plus, mécaniquement plus fiable.

Cette rotation est engendrée au moyen d'une plate-forme qui tourne (2), pourvue de moyens (15) pour consentir un replacement dans la position initiale après avoir accompli un tour complet de 360°.

Pendant l'opération de relèvement il faut s'assurer que la personne sous enquête ne bouge pas, c'est-à-dire qu'elle ne change pas de position ou de volume.

Un temps de 10 secondes environ de révolution de la plate-forme (2) résulte être expérimentalement le juste compromis pour obtenir une vitesse angulaire non dangereuse pour l'individu, et en même temps être une période de temps soutenable aux fins de la constance de la position à garder.

Il en résulte, de plus, un temps de relèvement qui n'est pas trop long aux fins de l'utilisation du système pour des relevés répétitifs comme, par exemple, il pourrait être nécessaire dans les points de vente, dans les sièges de relèvement médical ou de recrutement militaire.

Afin d'analyser le contour du corps humain, il faut que le sujet à relever soit déshabillé ou en conditions d'habillement telles à ne pas fournir des images faussées dudit contour réel.

### Description de l'appareil

Les parties fondamentales qui constituent l'appareil sont :
- une télécaméra digitale (1)
- une plate-forme tournante (2)
- un ordinateur avec au minimum un Pentium Intel 333Mhz et 128 KB de RAM et Disque Dur de 2 Gigabyte (4)
- carte d'acquisition vidéo du type Grabber (5)
- carte graphique avec définition 1024x 740 pixels, à 65.000 couleurs (installée sur la carte mère de l'ordinateur)
- monitor couleur 17 pouces SVGA (7)
- imprimante graphique laser ou à jet d'encre (8)

La télécaméra (1) est progressif du type digital et équipé d'un optique composé des objectifs interchangeable en mesure de relever d'une distance de 2 mètres des individus appartenant à la bande de taille qui va de 600 millimètres jusqu' à 2200 millimètres. Cette ci est montée dans une position fixe à une distance d'approximativement 2 mètres du centre de la plateforme tournante et à une hauteur d'approximativement 165 centimètres de la terre et est reliée au moyen de deux câbles d'alimentation et transmission des données, à la carte spécifique (5) installée dans l'ordinateur.

L'estrade tournante (2), laquelle contient la motorisation (9) et les encodeurs (10) pour la commande des passes de rotation, est commandé des cartes et des interrupteurs mis sur un panneau de commande (11) qui est placé, généralement, près de l'ordinateur (4).

L'estrade tournante (2) également est équipé des photodetecteurs (15) qui permettent par un module (6) de signaler au panneau de commande la position de la référence (initiale et à repos) de la rotation du repose-pieds (2). Le panneau de commande (11) informera la carte grabber (5) que l'estrade (2) est dans la position de référence 0 degrés (c'est à dire qu'elle a terminé 360 degrés de révolution complète). Au-dessus de la base de l'estrade sont situés deux formes moulées sous pression (12) pour loger et indiquer la position correcte des pieds et pour garantir la stabilité du corps pendant la rotation. À la même fonction de stabilité deux batons (13, 13') solidaires avec l'estrade tournante (2) qui se projettent en divergeant en haut, fournis de poignées (14,14') qui se vissent à differentes hauteurs en fonction de l'hauteur des sujets a relever dequ'ils facilitent un positionnement du corps pour l'étude en contraste et la position des bras espacés du corps et pliés de manière à former un angle apte à la mensuration de leurs longueurs.

Le panneau lumineux (3), ayant de préférence la dimension 250 cm x 1150 cm et distant 16 cm du sujet à relever, consiste généralement en un panneau de Plexiglas blanc opaque, derrière lequel sont installées cinq lampes au néon (15) de puissance totale égale à 175 Watt (35 x 5). La fonction dudit panneau est de fournir une surface à luminosité rigoureusement constante afin que les contours du corps humain puissent être relevés par la télécaméra (1) de façon suffisamment précise et contrastée ; il est évidemment possible d'adopter d'autres matériels qui remplissent la même fonction de contraste.

La précision et le contraste de l'image dépendent d'une situation de luminosité appropriée à l'environnement : une variante de la réalisation de la présente invention prévoit que le système de relèvement soit à l'intérieur d'une cabine à parois noires non réfléchissantes, c'est-à-dire de façon à ne pas fournir une illumination indirecte, par réflexion ou réverbération sur les parties du corps vues par la caméra. La cabine, non indiquée dans les figures, et qui entoure l'estrade tournante, peut être construite comme une structure mobile dans laquelle les parois sont amovibles, ou comme une installation fixe .

Les logiciels sont schématiquement composés de trois modules principaux :
A - logiciel de base
B - logiciel pour l'acquisition des images et pour l'élaboration des mesures.
C - logiciel pour l'attribution des conformations.

Le logiciel de base est le milieu opérationnel dans lequel les deux autres modules agissent. Dans la réalisation préférentielle de l'invention on utilise MS WINDOWS NT, où, pour le data base relationnel, pour réaliser la fonction de data management on utilise le programme ACCESS appartenant à la SUITE OFFICE 97 ; les mêmes opérations du procédé de la présente invention peuvent être évidemment effectuées par des systèmes opérationnels et des programmes différents de ceux que l'on a utilisés.

Le logiciel d'acquisition des images utilisées est une application avec des modules en langage C et en Assembler et utilise des librairies et des primitives de la carte d'acquisition spécialisée.

Le macro procédé peut être décrit par le flux suivant :
1) la télécaméra acquière les images du contour du corps et les envoie à la carte ;
2) la carte mémorise les 92 silhouettes et les rend disponibles à l'élaboration du module logiciel suivant ;
3) ledit module calcule le périmètre des ellipsoïdes correspondants aux sections des plans transversaux à l'axe vertical du corps sous relèvement : le nombre et la position de telles sections transversales sont assignés par des considérations inhérentes au choix des mesures importantes pour la détermination des conformations, et en général, pour la construction d'une pièce d'habillement.

Par conséquent les sections fondamentales sont, par exemple dans le cas d'application au secteur de l'habillement, celles qui sont placées au point de taille, au bassin, à la poitrine (sous aisselle), au cou, etc.

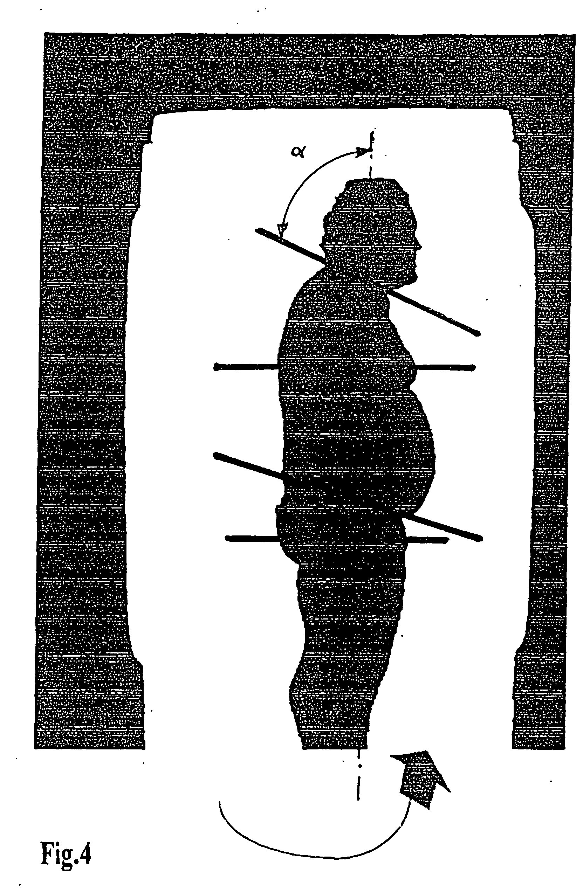

L'inclination de tels plans transversaux par rapport à l'axe longitudinal du corps peut varier d'une façon paramétrique et le logiciel calcule les ellipsoïdes correspondantes à l'intersection des plans obliques avec le corps du sujet sous relèvement : cela offre la possibilité d'obtenir des sections inclinées par rapport au susdit axe vertical et donc de permettre des mesures des parties complexes du corps humain (fig.4) dont les applications sont utiles dans le domaine de l'anthropologie médicale et de l'ergonomie.

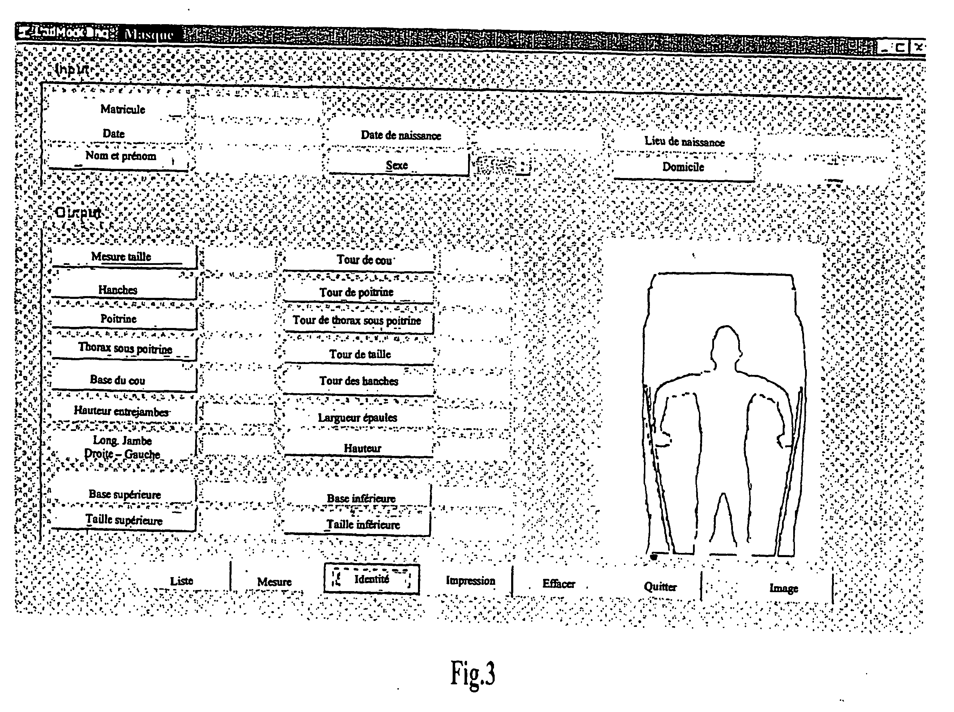

Le module logiciel peut également obtenir des mesures linéaires en calculant la longueur des segments relatifs à des vues frontales ou latérales déterminées du corps humain acquis comme images, comme dans les figures 3-4, en rendant possible les applications déjà divulguées en JP-A 6104430 et en EP 0524119.

Chacune des 90-92 vues est mémorisée et donc rendue disponible en forme statique au vidéo de l'ordinateur pour les enquêtes les plus disparates : pratiquement il est possible de visualiser une vue pour chaque 3,9° - 4° de rotation du corps.

La détermination de l'échelle de mesure à utiliser (réglage) est accomplie à travers une comparaison interne parmi les images de certaines distances relevées et d'autres connues à priori (par exemple, la distance entre les deux bâtons de soutien). Ce réglage est effectué seulement dans la phase d'essai ou dans le cas de coups accidentels ou de déplacements relatifs de la structure caméra-estrade tournante.

Le logiciel d'attribution des conformations est ce module qui, à partir des mesures des parties prééminentes du corps humain, permet d'assigner à l'anatomie relevée une classification selon les conformations anthropométriques mémorisées et une taille. Ces parties prééminentes sont le tour de poitrine, le tour de taille, le tour de hanches.

Le module permet de faire cette assignation de manière différenciée pour la partie inférieure et la partie supérieure du corps : il est donc possible d'avoir une anatomie à laquelle sera assignée une "conformation anthropométrique" et une taille à utiliser pour la construction d'une veste et une autre "conformation anthropométrique" /taille à utiliser pour la construction du pantalon ; les résultats du procédé de relèvement, de calcul et de comparaison avec la base de données des normotypes seront indiqués automatiquement sur le masque de la fig.3.

Lorsque les mesures relevées ne permettent pas d'associer une conformation anthropométrique à une taille, l'appareil signale la correspondance manquée.

Il est possible de varier la tolérance à utiliser pendant la recherche dans les tableaux des conformations, à travers l'assignation des valeurs paramétriques comme données d'entrée du programme. Lesdites tolérances sont liées à l'idée du bien-aller qu'on veut assigner à la pièce d'habillement qui sera projetée à partir des mesures relevées.

Deux éléments fondamentaux et caractérisants le procédé et l'appareil relatif doivent être mis en évidence :
le système de relèvement utilisé, qui consiste à capturer 92 points pour chaque plan transversal du corps, se révèle extrêmement effcace pour la reconstruction de la forme correcte du périmètre, soit en termes quantitatifs pour la précision de la mesure, soit en termes qualitatifs pour ce qui concerne la courbure et la forme de l'ellipsoïde. La précision de la mesure est de l'ordre de 2-3% et est aussi affectée par les déplacements imperceptibles mais toujours présents de l'individu pendant la révolution de 360°.

Une conséquence très importante de cette technologie est que par le relèvement du point sur lequel positionner le plan transversal et l'inclinaison de ce plan, il est possible d'avoir immédiatement la mesure de n'importe quel profil dont la silhouette est visible par la caméra, en contraste avec le panneau lumineux. Par rapport à des mesures tirées de l'élaboration de deux seules vues du corps humain, comme en EP 0524119, la frontale et la latérale, le système fournit des mesures conformes au volume relatif : en effet, en partant de deux seules vues la reconstruction du périmètre d'une section est possible seulement à travers des approximations ou du type paramétrique ou par comparaison avec des modèles anthropomorphiques prémémorisés, qui doivent être reconnus conformes au modèle relevé et retenus, pour cette raison, comparables. Il est évident qu'on pouvait avoir la possibilité d'obtenir un grand nombre de vues aussi bien, comme opté, avec une télécaméra fixe et le sujet en mouvement, qu'avec le sujet fixe et la caméra en mouvement, ou encore avec plusieurs caméras pour toutes les vues à numériser.

Le choix de réalisation de la présente invention a été dicté par des compromis entre la complexité mécanique et la garantie du procédé de relèvement et de l'appareil qui le réalise.

En effet, garder le sujet fixe et faire tourner la télécaméra auraient comporté que le panneau de contraste avec son équipement d'illumination tourne d'une façon solidaire à ladite caméra, qui, à son tour, est branchée avec des câbles à l'ordinateur : ledit choix ne résulte pas être optimal pour d'autres raisons que celles citées de complexité mécanique, parmi lesquelles, par exemple, parce ce qu'on obtiendrait une profondeur de l'image variable pendant le relèvement avec une conséquente imprécision du contour.

Soit dans le but d'une classification statistique, soit dans le but, fondamental, pour lequel le système a été inventé - projet d'une pièce d'habillement apte à habiller correctement le sujet qu'elle couvrira - la possibilité d'avoir une comparaison et une assignation de l'anatomie avec des classes morphologiques du type statistique est fondamental pour obtenir des résultats concrètement utilisables. En effet, si d'un coté il faut s'attendre dans le futur proche à une série de propositions commerciales parmi les plus diverses et technologiquement intéressantes en termes de simples systèmes de relèvement des mesures, cela ne répond pas , cependant, à la possibilité de réaliser correctement une pièce d'habillement à moins de ne pas fournir des informations du type quantitatif soit pour les volumes soit pour les proportions parmi les parties de tels volumes : en effet, c'est ce type d'indication qui est fourni par la classification statistique basée sur les conformations anthropométriques qui forment la base de données de référence du procédé de l'invention.

Il faut souligner que la présente invention fournit aussi une solution méthodologique dans la mesure où les valeurs numériques associées aux volumes à la base de la classification sont sujets aux variations naturelles et physiologiques et géographiques : l'invention consent, aussi, la mise à jour des valeurs numériques nécessaires à apporter les variations anthropométriques au modèle interne utilisé (base de données) et à fournir, donc, un procédé et un appareil pour la mis à jour statistique au moyen d'une simple introduction de nouvelles données dans la base de données anthropométrique.

Tout autant fondamental aux fins de l'unicité et de la validité conceptuelle de l'invention est la relation univoque existant entre les conformations anthropométriques (quelles qu'elles soient) et les bases bidimensionnelles qui constituent une pièce d'habillement dans laquelle ladite relation respecte les caractéristiques suivantes :
- un groupe de mesures du corps humain ne fournit pas les informations suffisantes pour réaliser une pièce d'habillement correcte du point de vue du bien-aller du vêtement, quel que soit le degré de précision de telles mesures : la respiration, la position assumée, le moment de la journée, le modèle de soutien-gorge porté sous une robe, etc..., sont des éléments qui changent les dimensions du corps (tour de taille, tour de poitrine...) de plusieurs cm. Si on ajoute à cela l'élasticité du tissu, on en déduit que pour la construction correcte d'une pièce d'habillement sont essentielles non seulement les mesures (entre des limites larges de tolérance) mais surtout la distribution de telles mesures le long du volume, c'est-à-dire la "forme" à laquelle elles donnent origine.
- II est donc nécessaire, pour fabriquer un vêtement qui habille correctement, de relever la distribution desdites mesures le long du corps, afin d'individualiser une forme. En utilisant, ensuite, les compétences de modélisme et les technologies pour construire les bases bidimensionnelles d'une pièce d'habillement pour chaque conformation, on peut gérer de façon automatique et il est donc possible de codifier avec des moyens informatiques les opérations de variation en fonction des différentes hauteurs.

La présente invention se prête à différentes réalisations qui se fondent sur le même parcours inventif : il ne faut donc pas considérer la description de la réalisation préférentielle limitative, des variations étant possibles même en restant dans le domaine des spécifiques des revendications.

**TABLEAU I**

| **(mesures femmes)** | | | | | | |
|---|---|---|---|---|---|---|
| | Base 10 | Base 8 | Base 6 | Base 4 | Base 2 | Base 0 |
| Taille | 36 38 40 | 38 40 42 44 46 48 | 40 42 44 | 42 44 46 | 44 46 48 | 46 48 50 |
| Poitrine | 80 84 88 | 82 86 90 94 98 102 | 87 91 95 | 93 97 101 | 98 102 106 | 102 106 110 |
| Tour de taille | 57 61 65 | 60 64 68 72 76 80 | 67 71 75 755 | 74 78 82 | 81 85 89 | 87 91 95 |
| Hanches | 86 89 93 | 88 92 96 100 104 108 | 95 99 103 | 102 106 110 | 107 111 115 | 115 119 123 |

**TABLEAU II**

| **(mesures hommes)** | | | | | | |
|---|---|---|---|---|---|---|
| | Base 10 | Base 8 | Base 6 | Base 4 | Base 2 | Base 0 |
| Taille | 44 46 48 | 46 48 50 52 54 56 | 48 50 52 | 50 52 54 | 52 54 56 | 54 56 58 |
| Poitrine | 88 92 96 | 100 104 108 112 116 120 | 96 100 104 | 101 105 109 | 104 108 112 | 107 111 115 |
| Tour de taille | 70 74 78 | 75 79 83 87 91 95 | 87 91 95 | 94 98 102 | 103 107 111 | 110 114 118 |
| Hanches | 83 87 91 | 90 94 98 102 106 110 | 97 101 105 | 103 107 111 | 108 112 116 | 113 117 121 |

## Revendications

1. Procédé pour l'identification et la classification anthropométrique des volumes à usage civil et militaire basé sur la prise de silhouettes en contraste de sujets humains, lesdites silhouettes en contraste étant élaborées au moyen d'algorithmes de calcul automatique comprenant en succession d'une première phase de prise continue desdites silhouettes contrastées desdits sujets humains, lesdits sujets tournant à vitesse angulaire constante; d'une deuxième phase d'acquisition automatique d'un nombre de silhouettes échantillonnées en écarts réguliers de rotation, le nombre de silhouettes étant suffisant à la reconstruction précise de la forme du volume dudit sujet tournant, **caractérisé en ce que** le procédé comprend aussi une troisième phase de calcul des périmètres d'ellipsoïdes obtenus par l'intersection de plans passant à travers l'axe vertical et à différentes hauteurs dudit sujet tournant en position bien droite avec le volume dudit sujet tournant ; une quatrième phase d'élaboration desdits périmètres d'ellipsoïdes au moyen d'algorithmes aptes à établir des relations numériques entre eux afin d'identifier la forme du volume dudit sujet tournant ; une cinquième phase de comparaison desdites relations numériques avec les données mémorisées dans la base de données contenant la représentation numérique des conformations anthropométriques; une sixième phase d'assignation automatique des mesures linéaires tirées desdites ellipsoïdes et représentatives du système de tailles dudit sujet et sa classification automatique en classes en accord à ladite représentation numérique desdites conformations anthropométriques.

2. Procédé selon la revendication 1 **caractérisé en ce que** le nombre de silhouettes échantillonnées automatiquement à écarts réguliers de rotation varie de 90 à 92.

3. Procédé selon les revendications 1 et 2 **caractérisé en ce que** le sujet tournant achève une révolution dans un temps de l'ordre de 10 secondes.

4. Procédé selon les revendications 1 et 3 **caractérisé en ce que les** plans qui passent à travers l'axe vertical du sujet tournant en position bien droite sont normaux au dit axe vertical.

5. Procédé selon les revendications 1-3 **caractérisé en ce que** les plans qui passent à travers l'axe vertical du sujet en position bien droite sont paramétriquement inclinés par rapport au dit axe vertical.

## Patentansprüche

1. Verfahren zur anthropometrischen Identifizierung und Klassifizierung der Umfänge für Zivil- und Militärgebrauch, das auf dem Vergleich von menschlichen Figuren basiert; solche Kontrast bildende Figuren werden über automatische Algorithmusrechnungen erstellt, die verschiedene aufeinanderfolgende Phasen umfasst: eine erste, kontinuerliche Vergleichsphase aller Figuren der genannten Menschen, die sich bei einer konstanten Winkelgeschwindigkeit drehen müssen; eine zweite Phase zur automatischen Gewinnung einer Figurenmenge, die als Muster nach regulären Drehintervallen aufgenommen wurde. Die Figurenmenge muss für die genaue Wiederherstellung der Umfangsform des sich drehenden Menschen genug sein; Das Verfahren ist **dadurch gekennzeichnet, dass** es eine dritte Phase der Errechnung von Elypsaidumfängen umfssst, die ihrerseits aus dem Schnitt der durch die vertikale Achse verlaufenden Ebenen und zu verschiedenen Höhen des völlig aufrecht stehenden und drehenden Menschen gewonnen wurden; eine vierte Phase zur Ausarbeitung solcher Elypsoidparameter über passende Algorithmen, die zu einer Bestimmung der numerischen Beziehungen zwischen ihnen und zur Identifizierung der Umfangsform des sich drehenden Menschen führen; in einer fünften Phase werden solche numerische Beziehungen mit den auf der Datenbank, die die numerische Darstellung der anthropometrischen Konfigurationen enthält, gespeicherten Angaben verglichen. Eine sechste Phase besteht aus der automatischen Zuordnung der aus den Elypsoiden erhaltenen und für das Größensystem dieses Menschen repräsentativen Linearabmessungen sowie aus der automatischen Klassifizierung in Klassen gemäß der numerischen Darstellung solcher anthropometrischen Konfigurationen.

2. Verfahren nach den Patentanspruch 1 **gekennzeichnet dadurch, dass** die Menge der als Muster automatisch und zu regulären Drehintervallen aufgenommenen Figuren zwischen 90 und 92 schwankt.

3. Verfahren nach den Patentansprüchen 1 und 2 **gekennzeichnet dadurch, dass** der sich drehende Mensch eine Drehung in einer sich in der Höhe von 10 Sekunden erstreckenden Zeit vervollständigt.

4. verfahren nach den Patentansprüchen 1 und 3 **gekennzeichnet dadurch, dass** die Ebenen, die durch die vertikale Achse des sich völlig aufrecht drehenden Menschen verlaufen, in Bezug auf die genannte vertikale Achse normal sind.

5. Verfahren nach den Patentansprüchen 1 und 3 **gekennzeichnet dadurch, dass** die Ebenen, die durch die vertikale Achse des völlig aufrecht drehenden Menschen verlaufen, in Bezug auf die genannte vertikale Achse parametrisch geneigt sind.

## Claims

1. Method for anthropomorphic identification and classification of human body volume for military and civilian purposes, based in taking a number of contrasted silhouettes of human subjects, said contrasted silhouettes being elaborate by means of automatic calculation algorithms including in succession a first phase of continuous taking of said contrasted silhouettes of said human subjects, said subjects turning at constant angular speed: a second phase of automatic acquisition of a number of silhouettes sampled at regular interval of rotation, the number of silhouettes being sufficient to a precise reconstruction of the shape of the volume proe of said turning subject, **characterized in that** the method includes also a third phase of calculation of the ellipsoids perimeters obtained by the intersection of plans passing through the vertical axis and at various heights of said subject turning in standing up position with the volume of the same turning subject; a fourth phase of elaboration of said ellipsoids perimeters by means of algorithms apt to establish numerical relations between them in order to Identify the shape of the volume of said turning subject; a fifth phase of comparison of said numerical relationships with the memorized data in the data base containing the digital representation of anthropometric conformations; a sixth phase of automatic assignment of linear measurements obtained by means of said linear ellipsoids and representing the system of sizes of said subject and its automatic classification in classes according to said digital representation of said anthropometric conformations.

2. Method according to claim 1 **characterized in that** the number of silhouettes automatically sampled at regular interval of rotation varies from 90 to 92.

3. Method according to claims 1 and 2 **characterized in that** the turning subject completes a revolution in a time of about 10 seconds.

4. Method according to claims 1 and 3 **characterized in that** the plans which pass through the vertical axis of the turning subject in standing up position are perpendicular to said vertical axis.

5. Method according to claims 1-3 **characterized in that** the plans which pass through the vertical axis of the subject in standing up position are parametrically tilted compared to said vertical axis.
